(19) **Europäisches Patentamt**
**European Patent Office**
**Office européen des brevets**

(11) **EP 1 351 736 B1**

(12) **EUROPEAN PATENT SPECIFICATION**

(45) Date of publication and mention
of the grant of the patent:
**14.05.2008 Bulletin 2008/20**

(21) Application number: **01271238.6**

(22) Date of filing: **18.12.2001**

(51) Int Cl.:
**A61M 25/01** *(2006.01)* **G01R 33/28** *(2006.01)*
**A61B 5/06** *(2006.01)*

(86) International application number:
**PCT/GB2001/005628**

(87) International publication number:
**WO 2002/049705 (27.06.2002 Gazette 2002/26)**

(54) **MAGNETIC FIELD GENERATING SYSTEM AND METHOD**

MAGNETFELDGENERATOR UND VERFAHREN

PROCEDE ET SYSTEME DE PRODUCTION DE CHAMPS MAGNETIQUES

(84) Designated Contracting States:
**DE FR GB IT NL**

(30) Priority: **21.12.2000 GB 0031287**

(43) Date of publication of application:
**15.10.2003 Bulletin 2003/42**

(73) Proprietor: **OXFORD INSTRUMENTS PLC**
**Witney,**
**Oxon OX8 1TL (GB)**

(72) Inventor: **Hanley, Peter**
**Gloucestershire GL15 4JQ (GB)**

(74) Representative: **Skone James, Robert Edmund**
**Gill Jennings & Every LLP**
**Broadgate House**
**7 Eldon Street**
**London EC2M 7LH (GB)**

(56) References cited:
**WO-A-99/23946**     **WO-A-99/60370**
**DE-A- 4 014 947**     **US-A- 5 592 939**

**Description**

The present invention relates to a magnetic field generating system, particularly for use in steering a catheter.

Catheterisation is a common procedure in which a catheter is inserted into the body of a subject such as a human or animal for performing a variety of further procedures. The catheter is urged through body cavities and lumens such as blood vessels in order to reach a treatment position such as the site of an aneurism. It has particular advantages over more traditional methods of open surgery as the trauma to the subject is significantly reduced.

Traditional methods of steering catheters involve the provision of a bent tip at the front end of the catheter. By applying a torque about the axis of a catheter, the orientation of the bent tip can be used to guide the catheter along a tortuous path within the subject. This torque is conventionally applied by the surgeon performing the procedure. However, in many cases the torque required is large which causes the surgeon to become fatigued and the associated forces involved increase the risk of internal damage of the subject.

More recently a less traumatic method of steering catheters has been developed which involves the use of a magnetic catheter steered in accordance with an applied magnetic field. By controlling the magnitude and direction of the applied magnetic field, it is possible to steer the catheter within the subject by producing a resultant force on the catheter. Stereotaxis Inc. has developed a system using this technique and an example is described in WO99/11189 and WO99/23946.

A major problem with known apparatus of this type is that the external magnets for applying the magnetic field are often large and are positioned in a manner which prevents ease of use of the system with other equipment such as X-ray imaging devices. The provision of a number of powerful magnets surrounding the subject can significantly restrict the access of medical personnel to the subject during the procedure. The magnetic fields produced also extend over a large area and these "stray" fields may affect the operation of other equipment.

In accordance with a first aspect of the present invention, a magnetic field generating system for use in steering a catheter, comprises:

X, Y and Z magnetic field generators arranged to generate corresponding magnetic fields in mutually orthogonal X, Y and Z directions;

wherein each of the X and Y magnetic field generators comprises a pair of magnetic elements, each magnetic element having a polarity defining a magnetic axis, wherein the magnetic elements within each pair are arranged such that their magnetic axes are oriented in the Z direction in a substantially antiparallel manner with respect to one another, and wherein the magnetic elements are laterally spaced apart with respect to their magnetic axes,

the arrangement being such that the corresponding magnetic fields cooperate to generate a resultant magnetic field in a working region separated in the Z direction from the magnetic field generators.

Unlike in conventional systems, the present invention provides a working region that is separated from the magnetic field generators. The magnetic field generators are generally provided to one side of the working region rather than surrounding it. This enables the working region to be accessible by medical personnel and allows other medical equipment such as imaging devices to be brought into close proximity with the working region.

This is achieved in the case of the X and Y magnetic field generators by using magnetic elements arranged in an anti-parallel sense. The magnetic field lines can be imagined to exit from one element in a pair, pass through the working region and enter the corresponding element of the pair. Therefore, within the working region the magnetic field lines from the X and Y magnetic field generators are oriented substantially in the X and Y directions respectively.

Typically the magnetic fields will be generated by electromagnets, generally in the form of electrically conducting or superconducting coils. As an alternative, permanent magnets could be provided although these are more difficult to control. Therefore, typically the magnetic elements within a pair will comprise electrically conducting coils of similar dimensions having a similar number of turns such that the magnetic field produced by each will be of a similar configuration and strength. However, the electrical currents within these coils will be arranged to flow in an opposite manner so as to generate similar fields having opposed polarities. The magnetic field at the centre of each coil will therefore be equal and opposite to that at the centre of its counterpart, such that in each case the coils are effectively coupled in series opposition. In general, the coils in the X magnetic field generator will be dissimilar to those of the Y magnetic field generator.

The Z magnetic field generator may comprise a single magnetic element, typically also provided as a coil. The dimensions of this coil will generally differ from those of the X and Y magnetic elements. However, preferably the Z magnetic field generator will also comprise at least two Z magnetic elements, each having a polarity defining a magnetic axis, wherein the Z magnetic elements are arranged such that their magnetic axes are oriented in the Z direction in a substantially anti-parallel manner with respect to one another. Unlike in the X and Y magnetic field generators, the Z magnetic elements will preferably be arranged coaxially with respect to one another. However, unlike the coils of the X and Y magnetic field generators, they will generally differ in their dimensions with respect to one another.

The use of opposed coils in all cases is advantageous in that it reduces the strength of any resultant stray magnetic fields at locations far from the magnetic field generators. This in turn reduces the adverse effect of these magnetic fields on other equipment or objects. The likelihood of any interaction between these generated magnetic fields and those produced by other equipment is also reduced. As a result, greater localized magnetic field strengths can be used.

Each of the coils may be provided with a suitable magnetic core material, such as soft iron in which the magnetisation does not persist after the removal of the electrical current.

In accordance with a second aspect of the present invention we provide a catheter steering system comprising:

a magnetic field generating system according to the first aspect of the invention; and

a control system for controlling the magnetic field generators such that the resultant magnetic field may be controlled in order to steer a catheter positioned within the working region.

Typically the control system will comprise a processor preferably provided as part of a computer for controlling the magnetic field generators. The strengths of the magnetic fields produced by the magnetic field generators are controlled in each case so as to produce a resultant magnetic field having the desired direction and strength.

An associated store, input device and display will be generally provided in association with the processor. If the magnetic fields are to be provided using electromagnets, then preferably the system will also include an electrical signal generator for supplying appropriate electrical signals to the magnetic field generators.

In many cases a non-magnetic support will be provided for supporting the body of a subject and although this may take the form of a seat, typically the support will be planar such as a table and will be positioned between the magnetic field generators and the working region. Such a support may be relatively movable along one or more axes with respect to the magnetic field generators in order to position the catheter correctly with respect to the resultant magnetic field. In general however, the working region will be of a sufficient extent such that relative movement between the support and the magnetic field generators will not be required.

Preferably the magnetic field generators will be arranged such that the resultant magnetic field within the working region will be substantially uniform, that is substantially constant in magnitude and direction, along at least one axis and preferably along two or three axes.

We also provide a method of operating a catheter steering system according to the second aspect of the invention, the method comprising:

locating a catheter within the working region; and
controlling the magnetic field generators using the control system such that the catheter is steered by a magnetic interaction with the resultant magnetic field.

Some examples of a magnetic field generating system will now be described with reference to the accompanying drawings, in which:-

Figure 1 shows an X magnetic field generator according to a first and second example;
Figure 2 shows the X magnetic field generator of Figure 1 with the addition of a Y magnetic field generator, according to the first and second examples;
Figure 3 illustrates the dimension measurements for the coils;
Figure 4 is a graph of the magnetic fields and magnetic field gradients for the X coils as a function of X displacement;
Figure 5 is a graph of Bz for the X coils as a function of X displacement;
Figure 6 is a graph of the magnetic fields and magnetic field gradients for the Y coils as a function of Y displacement;
Figure 7 is a graph of the magnetic fields and magnetic field gradients for the Y coils as a function of Z displacement;
Figure 8a illustrates the X,Y and Z magnetic field generators according to the first example;
Figure 8b is a schematic plan view of the X, Y and Z magnetic field generators according to the first example;
Figure 9 is a graph of the magnetic fields and magnetic field gradients for the Z coils of the first example as a function of X displacement;
Figure 10 is a graph of the magnetic fields and magnetic field gradients for the Z coils of the first example as a function of Z displacement;
Figure 11 shows the magnetic field for the X coils as a function of Z and X displacement;
Figure 12 shows the magnetic field for the Z coils of the first example as a function of Z and X displacement;
Figure 13 illustrates the X,Y and Z magnetic field generators according to the second example;
Figure 14 is a graph of the magnetic fields and magnetic field gradients for the Z coils of the second example as a function of Z displacement;
Figure 15 shows the magnetic field for the Z coils of the second example as a function of Z and X displacement;
Figure 16 is an illustration of a catheter control system according to the second example; and

Figure 17 is a flow diagram of a method of operating the catheter control system of the first or second examples.

An example of a system and method of catheter steering will now be described. The objective is to produce a magnetic flux density of 0.5 Tesla in any direction within the working region, this flux density being sufficient to steer a magnetic catheter within a subject.

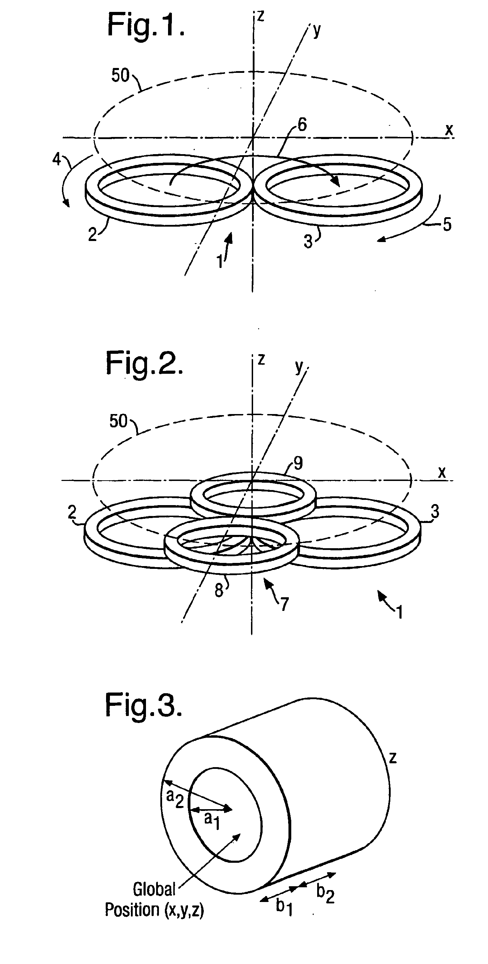

Figure 1 illustrates an "X" magnetic field generator 1 for generating a magnetic field in a direction X within a working region 50. The X magnetic field generator 1 comprises two electrically conducting annular coils 2,3 positioned adjacent one another with the centres of the coils aligned parallel to the X direction. The coils 2,3 are arranged such that a single plane bisects them, the plane having a normal that is parallel to the axes of the coils. Each coil is of similar dimensions.

The coils 2,3 each comprise a similar number of turns of electrically conducting or superconducting wire arranged such that in use, electrical currents flow within the coils 2,3 in the opposing directions marked 4,5. Each coil therefore acts as a magnet having north and south poles arranged along the Z axis (normal to the plane of the coils as indicated in Figure 1). Due to the opposing current directions in the coils, the combined magnetic field produced above the coils is generally represented by the arrow 6. The magnetic fields of the two coils are of similar strength and configuration but opposite in polarity.

Figure 2 shows the addition of a Y magnetic field generator 7 which, in a similar manner to the X magnetic field generator, comprises two opposing current coils 8,9 arranged along the Y axis. The centres of the coils of the X and Y magnetic field generators 1,7 therefore lie in an X-Y plane. It should be noted that the coils 8,9 are of slightly smaller diameter than the coils 2,3.

The dimensions of the coils 2, 3, 8, 9 are given in Table 1 along with their coordinates with respect to the orthogonal axes shown in the Figures 1 and 2. In the Table, values for a1,a2,b1,b2,X,Y,Z are given in metres, and the current density is given in amperes per square metre. As is shown in Figure 3, the measurements a1 and a2 represent internal and external radii of the coils, whereas b1 and b2 indicate the thickness of the coils along their major axes relative to the X-Y plane.

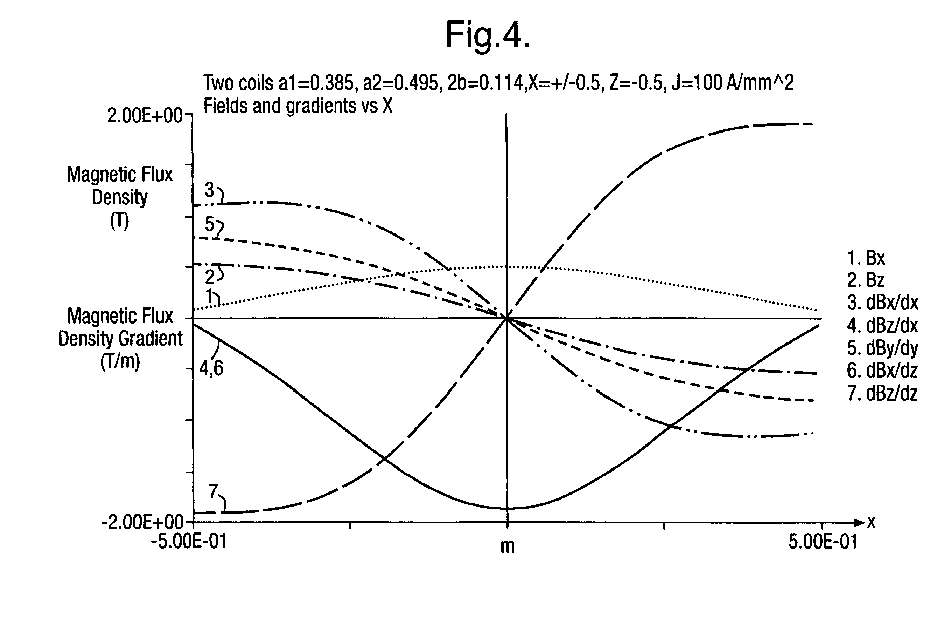

Figure 4 shows the magnetic field components and corresponding magnetic field gradient components for the X magnetic field generator in isolation as represented in Figure 1 and positioned in a plane 0.5 m below the origin of the co-ordinate axes. As the curve "1" indicates, the component of the field in the X direction varies only slightly over an extended displacement along the X coordinate axis. There are also no field components along the Y and Z directions due to the symmetry but it should be noted that there is a substantial gradient $dB_x/dz$ as is to be expected. To conserve flux (i.e. to obey the divergence theorem) there is an equally large gradient $dB_x/dx$.

The magnetic field gradients will produce a force on the magnetic catheter but this force will be relatively minor. These gradients are a consequence of magnetic flux continuity and it is desirable that within the working region they do not significantly affect the magnetic catheter.

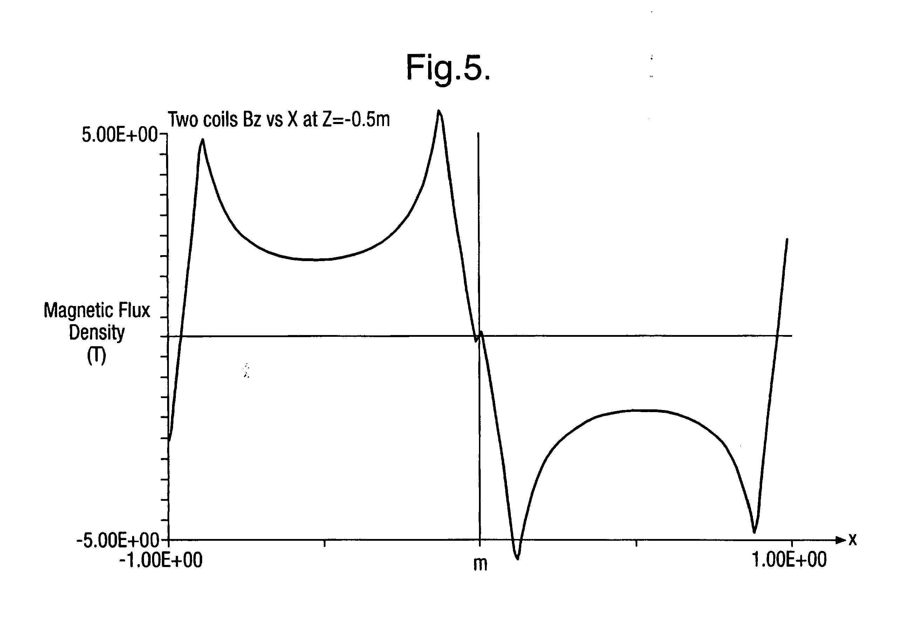

Figure 5 shows the variation in the magnetic field component along the Z axis as a function of displacement along the X axis at Z=-0.5 m, that is passing through the X coils 2,3. The peak magnetic field is approximately 5 Tesla and therefore the assumed current density of $10^8$ Am$^{-2}$ is a realistic value for the desired application.

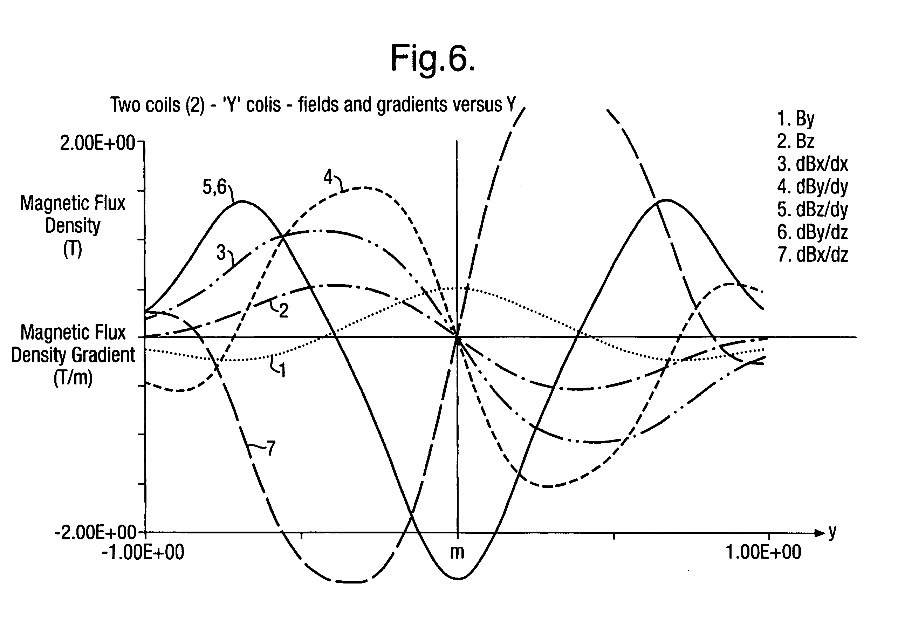

Figure 6 shows the fields and gradients for the Y magnetic field generator as a function of the displacement along the Y axis. Again the magnetic field component in the X direction along this axis is zero for this particular magnetic field generator due to the symmetry.

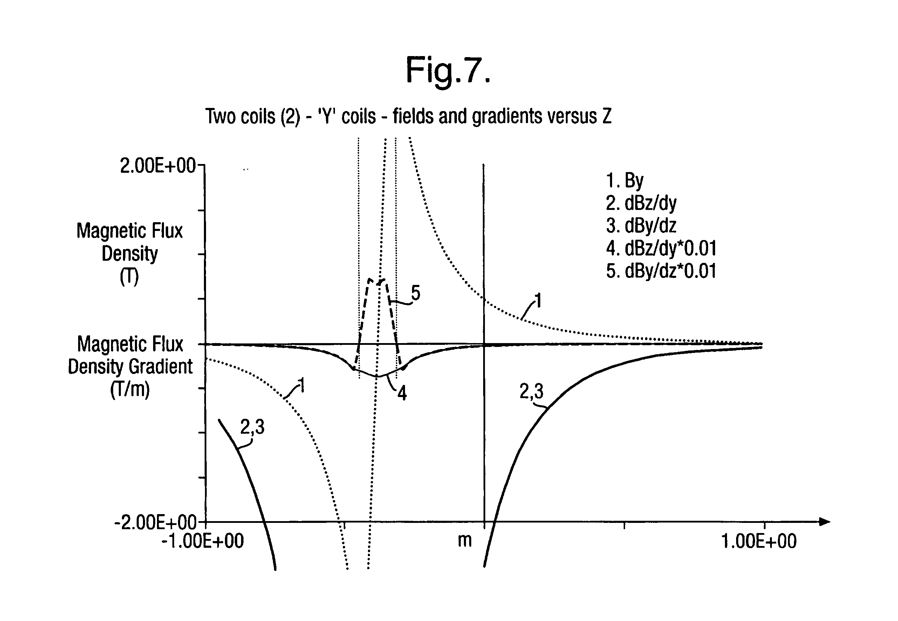

Figure 7 shows the magnetic fields and magnetic field gradients for the Y coils along the Z axis. The Y coils are located at +/-0.375 metres respectively.

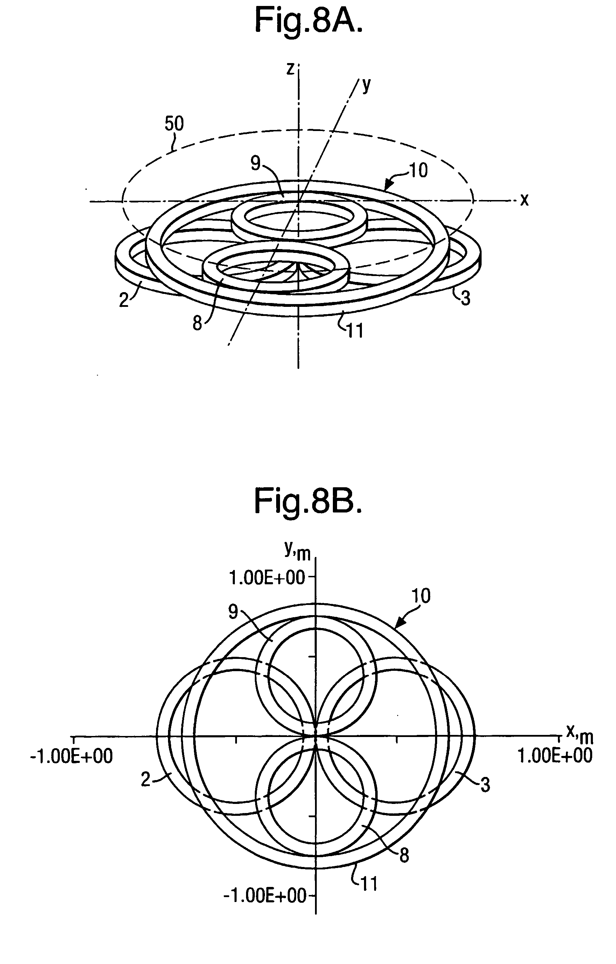

A Z magnetic field generator 10 in accordance with a first example of the invention is shown in Figures 8a and 8b. The Z magnetic field generator comprises a single electrically conducting coil 11 of larger diameter than the coils in the X and Y magnetic field generators. It is arranged to encircle the Y magnetic field generator coils and to have an axis parallel to those of the Y coils.

**TABLE 1**

| | | | | | |
|---|---|---|---|---|---|
| Coil 2 | Current density | 1.0000E+08 | X | -5.0000E-01 |
| | a1 | 3.8500E-01 | Y | 0.0000E+00 |
| | a2 | 4.9500E-01 | Z | -5.0000E-01 |
| | b1 | -5.7000E-02 | | |
| | b2 | 5.7000E-02 | | |
| | | | | |
| Coil 3 | Current density | -1.0000E+08 | X | 5.0000E-01 |
| | a1 | 3.8500E-01 | Y | 0.0000E+00 |

(continued)

|  |  |  |  |  |
|---|---|---|---|---|
| a2 | 4.9500E-01 | Z | -5.0000E-01 |
| b1 | -5.7000E-02 |  |  |
| b2 | 5.7000E-02 |  |  |

Y - coils:

| Coil 8 | Current density | 1.0000E+08 | X | 0.0000E+00 |
|---|---|---|---|---|
|  | a1 | 2.887SE-01 | Y | -3.7500E-01 |
|  | a2 | 3.7125E-01 | Z | -3.7500E-01 |
|  | b1 | -5.7000E-02 |  |  |
|  | b2 | 5.7000E-02 |  |  |

| Coil 9 | Current density | -1.0000E+08 | X | 0.0000E+00 |
|---|---|---|---|---|
|  | a1 | 2.8875E-01 | Y | 3.7500E-01 |
|  | a2 | 3.7125E-01 | Z | -3.7500E-01 |
|  | b1 | -5.7000E-02 |  |  |
|  | b2 | 5.7000E-02 |  |  |

Table 2 shows the respective current, dimension and coordinate data for this coil.

**TABLE 2**

| Coil 11 | Current | 1.0000E+08 | X | 0.0000E+00 |
|---|---|---|---|---|
|  | a1 | 7.5000E-01 |  | Y0.0000E+00 |
|  | a2 | 8.2500E-01 | Z | -3.7500E-01 |
|  | b1 | -5.7000E-02 |  |  |
|  | b2 | 5.7000E-02 |  |  |

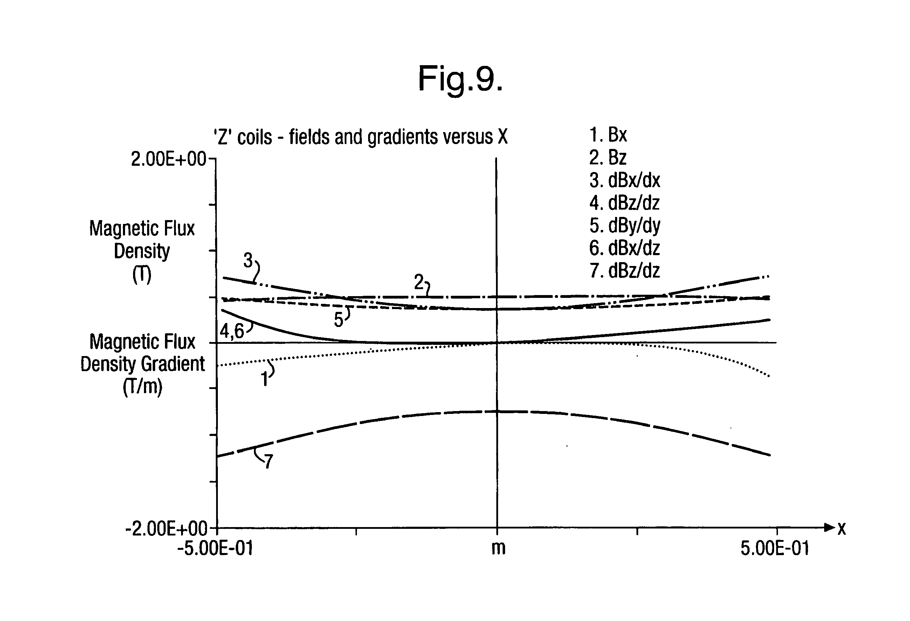

The magnetic field and magnetic field gradient produced by the coil 11 are indicated in Figure 9 with the coil 11 located at 0.375 metres below the coordinate axis. This coil produces a magnetic field having substantially uniform X and Z components as a function of displacement along the X axis.

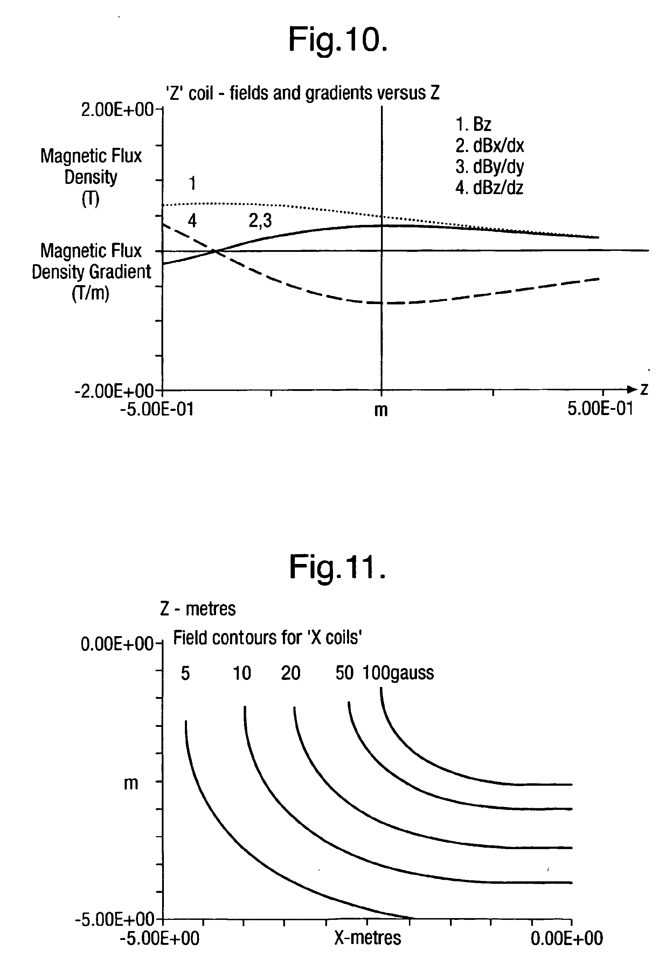

Referring to Figure 10, it can be seen that the Z component of the magnetic field due to the coil 9 reduces slowly as a function of distance from the Z coil. This is due to the use of a single coil having a relatively large diameter.

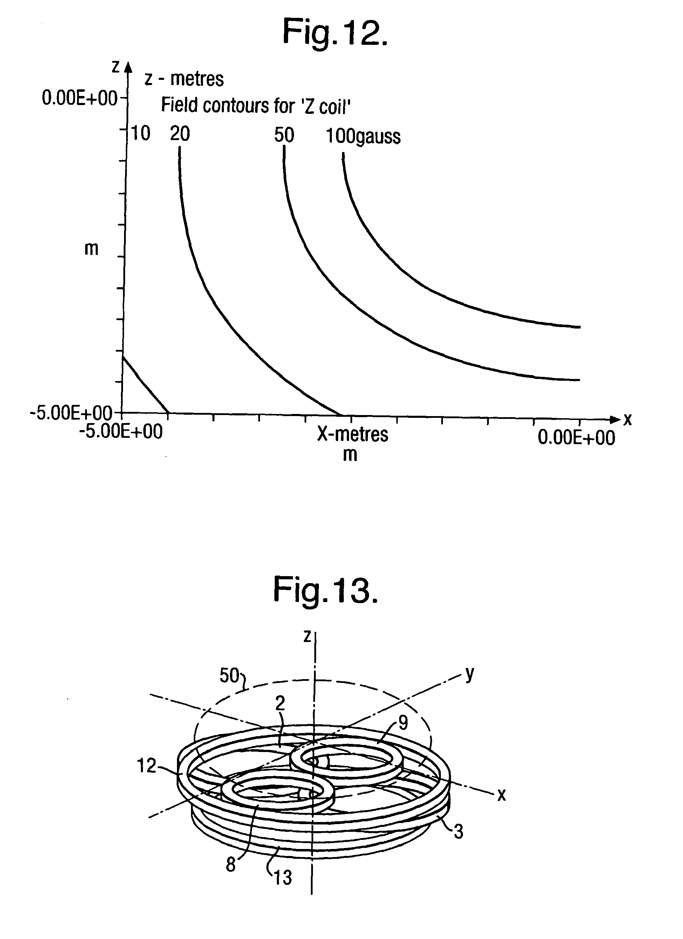

Figures 11 and 12 show the "stray" magnetic fields of the X coils and Z coils respectively as a function of the X and Z directions. The stray field due to the X coils (and Y coils) is considerably less than that due to the Z coils. This is because of the use of opposed coils in the X and Y magnetic field generators 1,7.

In a second example shown in Figure 13, a two opposed coil arrangement 12,13 is provided for the Z magnetic field generator 10. The coils of the X and Y magnetic field generators 1,7 are denoted by similar numerals. Two coils 12,13 are positioned so as to share a common axis along the Z direction, the upper coil 12 being of slightly larger diameter than the lower coil 13. The current density, dimensions and position of the coils 12,13 are shown in Table 3.

**TABLE 3**

| Coil 12 | Current | 1.0000E+08 | X | 0.0000E+00 |
|---|---|---|---|---|
|  | a1 | 7.5000E-01 | Y | 0.0000E+00 |
|  | a2 | 9.5000E-01 | Z | -3.7500E-01 |
|  | b1 | -6.7000E-02 |  |  |
|  | b2 | 5.7000E-02 |  |  |

| Coil 13 | Current | -1.0000E+08 | X | 0.0000E+00 |
|---|---|---|---|---|
|  | a1 | 6.2700E-01 | Y | 0.0000E+00 |
|  | a2 | 8.2700E-01 | Z | -8.0000E-01 |
|  | b1 | -7.8000E-02 |  |  |
|  | b2 | 7.8000E-02 |  |  |

The coils 12,13 are arranged to meet the following criteria:-

a) the net magnetic moment is zero in order to minimize the stray field; and,
b) the Z gradient in the region of interest is cancelled to give a large working region.

If the coils can be approximated as thin hoops, the dimensions which satisfy these conditions can be found. The radii of the two coils are $a_1$ and $a_2$, their axial positions are $b_1$ and $b_2$, and the ratio of ampere-turns of coil 2 to coil 1 is N:-

$$\pi a_1^2 + N \pi a_2^2 = 0 \quad \text{no net magnetic moment}$$

$$\frac{\theta B(a_1, b_1, z)}{\theta z} + N \frac{\theta B(a_2, b_2, z)}{\theta z} = 0 \quad \text{no z-gradient.}$$

These can be solved to give

$$\frac{a_2}{a_1} = \frac{b_2^{1/h} \sqrt{a_1^2 + b_1^2 - b_2^{2/h} \cdot b_1^{h/h}}}{a_1 \cdot b_1^{1/5}}, \qquad N = \frac{a_1^2}{a_2^2}$$

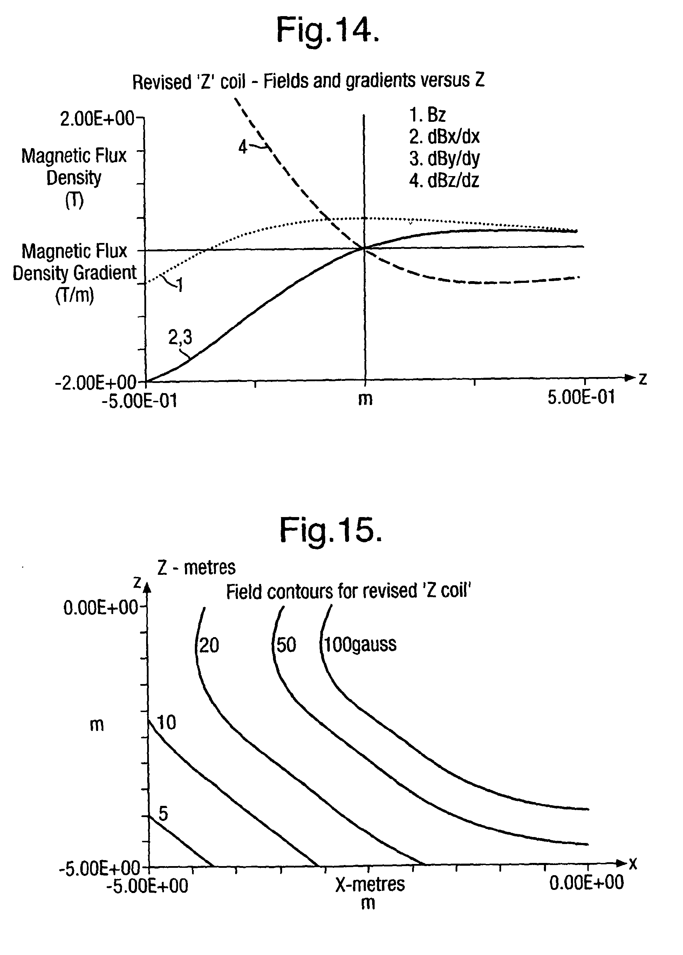

The magnetic field and magnetic field gradient for this arrangement are shown in Figure 14 with the corresponding field as a function of the X direction shown in Figure 15.

When Figure 15 is compared with Figure 12 a much more uniform field is produced and there is a reduction in the magnetic flux density of the stray field.

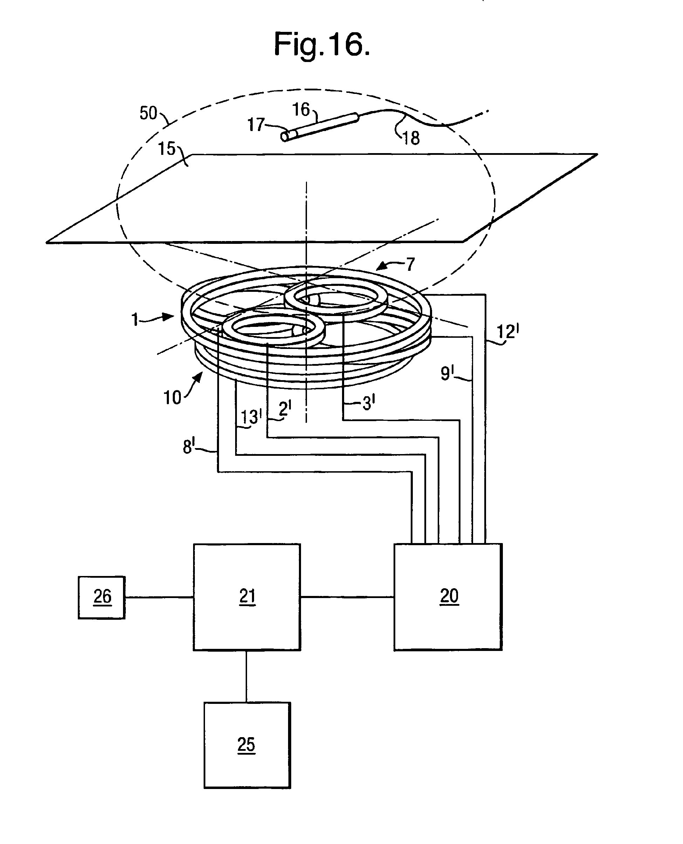

Figure 16 shows a magnetic catheter steering system incorporating the arrangement of magnetic field generators according to the second example, although those of the first example could be equally used. The X,Y and Z magnetic field generators 1,7,10 are positioned beneath a support 15 such as a bed or table, allowing approximately 0.25 metres of clear space above the coils and their housings. A catheter 16 is schematically represented above the support 15, the catheter 16 having a magnetic tip 17. The magnetic tip 17 is flexibly coupled to the body of the catheter 16 and may be caused to bend away from axial alignment with the catheter 16 in response to an applied magnetic field. In use, the catheter 16 is inserted within the body of a subject (not shown) lying on the support 15 and is urged in an axial direction using a guide wire 18.

Each of the coils 2,3,8,9,12,13 is connected to an electrical signal generator 20 with corresponding control lines 2',3',8',9',12',13'. Each of the coils in this example comprises superconducting wire. A suitable cooling system (not shown) is provided to maintain the coils at a superconducting temperature. The electrical signal generator 20 supplies electrical signals to the coils in response to instructions from a computer 21. The computer 21 has a processor along with an internal store for retaining the operating program code and parameters for use in controlling the coils.

A display 25 is used to display information to the operator of the system such as a surgeon and a number of input devices 26 such as a joystick, mouse and keyboard allow the surgeon to control the system.

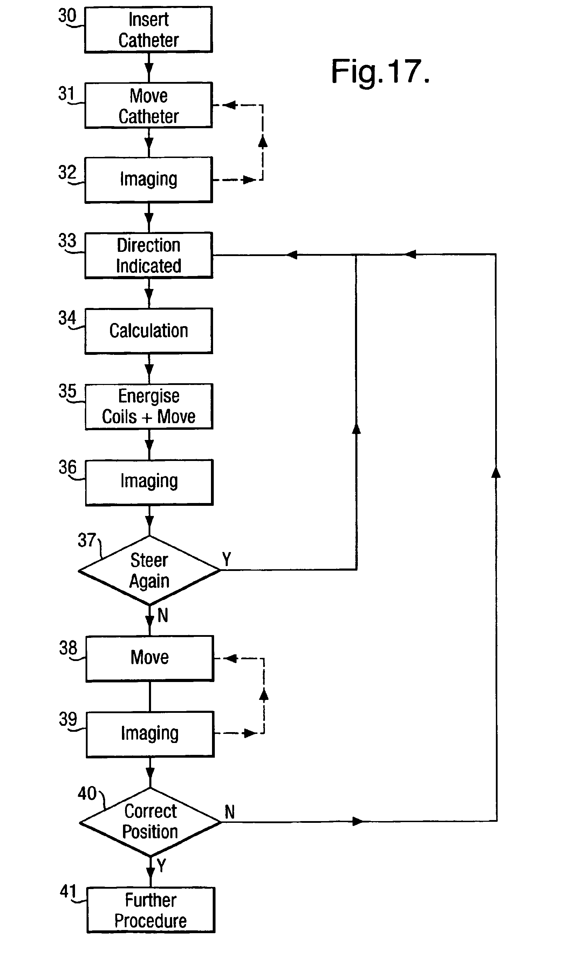

A method of operating the catheter steering system detailed above will now be described in association with Figure 17.

At step 30, the catheter 16 is inserted within a human patient at a convenient point such as a femoral artery. The guide wire 18 is then used to urge the catheter 16 along this lumen at step 31. The progress of the catheter is then monitored using an imaging technique at step 32, This imaging step may be performed simultaneously. Steps 31 and 32 may be repeated a number of times.

When the catheter 16 has reached an arterial junction at step 33 the surgeon operates the input device 26 to indicate to the computer 21 the desired direction in which the catheter 16 should be steered.

At step 34 the computer uses the known parameters of the magnetic field generators 1,7,10 to calculate a suitable combination of electrical signals to supply to the magnetic field generators 1,7,10 for steering the catheter 16 in the required direction.

At step 35 the computer supplies control signals to the electrical signal generator 20 which accordingly produces electrical signals in the control lines 2',3',8',9',12',13'. The signals supplied to the coils typically comprise a coordinated combination of electric currents. These produce a corresponding magnetic field associated with each of the relevant coils. The individual magnetic fields combine to produce a resultant magnetic field. The magnetic tip of the catheter 17 interacts with this resultant magnetic field and changes its orientation with respect to the body of the catheter 16. Further urging of the catheter 16 using the guide wire 18 at step 35 then allows the catheter to be steered in the new direction.

A subsequent (or simultaneous) imaging process is then performed at step 36 to monitor the progress of the catheter 16.

At step 37, if further steering is required then the steps 33 to 36 may be repeated, otherwise subsequent movement and imaging steps 38 and 39 may be performed. At step 40, if the catheter 16 has reached the desired position then the catheter 16 is used for conventional procedures at step 41. If further movement or steering is required before performing step 41 then steps 33 to 39 may be repeated.

**Claims**

1.  A magnetic field generating system for use in steering a catheter, the system comprising:

    X (1), Y (7) and Z (10) magnetic field generators arranged to generate corresponding magnetic fields in mutually orthogonal X,Y and Z directions;

    wherein each of the X and Y magnetic field generators comprises a pair of magnetic elements (2,3,8,9), each magnetic element having a polarity defining a magnetic axis, wherein the magnetic elements within each pair are arranged such that their magnetic axes are oriented in the Z direction in a substantially antiparallel manner with respect to one another, and wherein the magnetic elements are laterally spaced apart with respect to their magnetic axes,

    the arrangement being such that the corresponding magnetic fields cooperate to generate a resultant magnetic field in a working region (50) separated in the Z direction from the magnetic field generators.

2.  A system according to claim 1, wherein the magnetic field generators comprise electromagnets.

3.  A system according to claim 2, wherein the electromagnets are electrically conducting coils.

4.  A system according to any of the preceding claims, wherein the Z magnetic field generator (10) comprises at least two Z magnetic elements (12,13), each having a polarity defining a magnetic axis, wherein the Z magnetic elements are arranged such that their magnetic axes are oriented in the Z direction in a substantially antiparallel manner with respect to one another, and wherein the Z magnetic elements are axially spaced apart with respect to their magnetic axes.

5.  A system according to claim 4, wherein each magnetic element of the Z magnetic field generator is an electromagnet in the form of an electrically conducting coil.

6.  A catheter steering system comprising:

    a magnetic field generating system according to any of claims 1 to 5; and
    a control system for controlling the magnetic field generators such that the resultant magnetic field may be controlled in order to steer a catheter positioned within the working region.

7.  A system according to claim 6, wherein the control system includes a processor for controlling the magnetic field generators.

8.  A system according to claim 7 and when dependent upon at least claim 2, wherein the control system further comprises an electrical signal generator (20) for supplying the electromagnets with electric signals under the control of the processor.

9.  A system according to any of claims 6 to 8, further comprising a support (15) for supporting a subject for catheterisation, wherein the support is positioned between the magnetic field generators and the working region.

10. A system according to any of the preceding claims, wherein the magnetic field of at least one of the magnetic field generators is substantially uniform within the working region.

**Patentansprüche**

1.  Ein magnetfeldgenerierendes System zur Verwendung beim Lenken eines Katheters, wobei das System umfasst:

X(1), Y(7), Z(10) Magnetfeldgeneratoren, die angeordnet sind, um entsprechende Magnetfelder in zueinander senkrechten X-, Y- und Z-Richtungen zu generieren;

wobei jeder der X- und Y-Magnetfeldgeneratoren ein Paar magnetische Elemente (2, 3, 8, 9) umfasst, von denen jedes magnetische Element eine Polarität hat, die eine magnetische Achse definiert, wobei die magnetischen Elemente innerhalb jedes Paares derart angeordnet sind, dass ihre magnetischen Achsen in der Z-Richtung in einer im wesentlichen antiparallelen Weise in Bezug zueinander orientiert sind, und wobei die magnetischen Elemente seitlich in Bezug auf ihre magnetischen Achsen voneinander beabstandet sind, wobei die Anordnung derart ist, dass die entsprechenden Magnetfelder zusammenwirken, um ein resultierendes Magnetfeld in einem Arbeitsbereich (50) zu generieren, der in der Z-Richtung von den Magnetfeldgeneratoren getrennt ist.

2. System nach Anspruch 1, bei dem die Magnetfeldgeneratoren Elektromagnete umfassen.

3. System nach Anspruch 2, bei dem die Elektromagnete elektrisch leitende Spulen sind.

4. System nach einem der vorstehenden Ansprüche, bei dem der Z-Magnetfeldgenerator (10) wenigstens zwei Z-magnetische Elemente (12, 13) umfasst, von denen jedes eine Polarität hat, die eine magnetische Achse definiert, wobei die Z-magnetischen Elemente derart angeordnet sind, dass ihre magnetischen Achsen in der Z-Richtung in einer im wesentlichen antiparallelen Weise in Bezug zueinander orientiert sind, und wobei die Z-magnetischen Elemente axial in Bezug auf ihre magnetischen Achsen voneinander beabstandet sind.

5. System nach Anspruch 4, bei dem jedes magnetische Element des Z-Magnetfeldgenerators ein Elektromagnet in der Form einer elektrisch leitenden Spule ist.

6. Katheterlenksystem, umfassend:

ein magnetfelderzeugendes System nach einem der Ansprüche 1 bis 5; und
ein Steuersystem zum Steuern der Magnetfeldgeneratoren derart, dass das resultierende Magnetfeld gesteuert werden kann, um einen innerhalb des Arbeitsbereichs positionierten Katheter zu lenken.

7. System nach Anspruch 6, bei dem das Steuersystem einen Prozessor zur Steuerung der Magnetfeldgeneratoren aufweist.

8. System nach Anspruch 7 und abhängig von zumindest Anspruch 2, bei dem das Steuersystem ferner einen Generator (20) für ein elektrisches Signal umfasst, zum Versorgen der Elektromagnete mit elektrischen Signalen unter der Steuerung durch den Prozessor.

9. System nach einem der Ansprüche 6 bis 8, ferner umfassend einen Träger (15) zum Tragen eines für eine Katheterisierung vorgesehenen Subjekts, wobei der Träger zwischen den Magnetfeldgeneratoren und dem Arbeitsbereich positioniert ist.

10. System nach einem der vorstehenden Ansprüche, bei dem das Magnetfeld wenigstens eines der Magnetfeldgeneratoren innerhalb des Arbeitsbereichs im wesentlichen gleichförmig ist.

**Revendications**

1. Système de production d'un champ magnétique utilisé pour le guidage d'un cathéter, le système comprenant :

des générateurs de champ magnétique X (1), Y (7) et Z (10) agencés pour générer des champs magnétiques correspondants dans des directions X, Y et Z mutuellement orthogonales,

dans lequel chacun des générateurs de champ magnétique X et Y comprend une paire d'éléments magnétiques (2, 3, 8, 9), chaque élément magnétique présentant une polarité qui définit un axe magnétique, les éléments magnétiques de chaque paire étant agencés de manière à ce que les axes magnétiques soient orientés dans la direction Z d'une manière essentiellement antiparallèle les uns par rapport aux autres et les éléments magnétiques étant écartés latéralement par rapport à leur axe magnétique,
l'agencement étant tel que les champs magnétiques correspondants coopèrent pour générer un champ magnétique résultant dans une zone de travail (50) séparée des générateurs de champ magnétique dans la direction Z.

2. Système selon la revendication 1, dans lequel les générateurs de champ magnétique comprennent des électroaimants.

3. Système selon la revendication 2, dans lequel les électroaimants sont des bobines électriquement conductrices.

4. Système selon l'une quelconque des revendications précédentes, dans lequel le générateur (10) du champ magnétique Z comprend au moins deux éléments magnétiques Z (12, 13), chacun présentant une polarité qui définit un axe magnétique, dans lequel les éléments magnétiques Z sont agencés de telle sorte que leurs axes magnétiques soient orientés dans la direction Z d'une manière essentiellement antiparallèle les uns par rapport aux autres et dans lequel les éléments magnétiques Z sont espacés axialement les uns des autres par rapport à leur axe magnétique.

5. Système selon la revendication 4, dans lequel chaque élément magnétique du générateur de champ magnétique Z est un électroaimant qui présente la forme d'une bobine électriquement conductrice.

6. Système de guidage d'un cathéter comprenant :

   un système de production de champ magnétique selon l'une quelconque des revendications 1 à 5 et
   un système de contrôle qui contrôle les générateurs de champ magnétique de telle sorte que le champ magnétique résultant puisse être contrôlé pour guider un cathéter positionné dans une zone de travail.

7. Système selon la revendication 6, dans lequel le système de contrôle comprend un processeur qui contrôle les générateurs de champ magnétique.

8. Système selon la revendication 7 et selon au moins la revendication 2, dans lequel le système de contrôle comprend de plus un générateur (20) de signaux électriques qui délivre des signaux électriques aux électroaimants sous le contrôle du processeur.

9. Système selon l'une quelconque des revendications 6 à 8, comprenant de plus un support (15) qui supporte un patient sur lequel on doit placer un cathéter, le support étant positionné entre les générateurs de champ magnétique et la zone de travail.

10. Système selon l'une quelconque des revendications précédentes, dans lequel le champ magnétique d'au moins un des générateurs de champ magnétique est essentiellement uniforme dans la zone de travail.

## Fig.1.

## Fig.2.

## Fig.3.

Global
Position (x,y,z)

10

Fig.4.

Two coils a1=0.385, a2=0.495, 2b=0.114, X=+/-0.5, Z=-0.5, J=100 A/mm^2
Fields and gradients vs X

1. Bx
2. Bz
3. dBx/dx
4. dBz/dx
5. dBy/dy
6. dBx/dz
7. dBz/dz

Magnetic Flux Density (T)

Magnetic Flux Density Gradient (T/m)

2.00E+00

-2.00E+00

-5.00E-01

5.00E-01

x

m

# Fig.5.

Two coils Bz vs X at Z=-0.5m

EP 1 351 736 B1

# Fig.6.

Two coils (2) - 'Y' colis - fields and gradients versus Y

1. By
2. Bz
3. dBx/dx
4. dBy/dy
5. dBz/dy
6. dBy/dz
7. dBx/dz

EP 1 351 736 B1

# Fig.7.

Two coils (2) - 'Y' coils - fields and gradients versus Z

Legend:
1. By
2. dBz/dy
3. dBy/dz
4. dBz/dy*0.01
5. dBy/dz*0.01

Y-axis (upper): Magnetic Flux Density (T)
Y-axis (lower): Magnetic Flux Density Gradient (T/m)

Y-axis values: 2.00E+00, -2.00E+00

X-axis: -1.00E+00, m, 1.00E+00

# Fig.8A.

# Fig.8B.

# Fig.9.

'Z' coils - fields and gradients versus X

1. Bx
2. Bz
3. dBx/dx
4. dBz/dz
5. dBy/dy
6. dBx/dz
7. dBz/dz

EP 1 351 736 B1

# Fig.10.

'Z' coil - fields and gradients versus Z

1. Bz
2. dBx/dx
3. dBy/dy
4. dBz/dz

# Fig.11.

Field contours for 'X coils'

## Fig.12.

z - metres
Field contours for 'Z coil'

0.00E+00

10  20       50    100gauss

Z

m

-5.00E+00

-5.00E+00          X-metres          0.00E+00
                        m

X

## Fig.13.

# Fig.14.

Revised 'Z' coil - Fields and gradients versus Z

2.00E+00

Magnetic Flux
Density
(T)

Magnetic Flux
Density Gradient
(T/m)

-2.00E+00

-5.00E-01

m

5.00E-01

Z

1. Bz
2. dBx/dx
3. dBy/dy
4. dBz/dz

4

1

2,3

# Fig.15.

Z - metres

Field contours for revised 'Z coil'

Z

0.00E+00

m

-5.00E+00

20

50

100gauss

10

5

-5.00E+00

X-metres
m

0.00E+00

X

# Fig.16.

# Fig.17.

30 Insert Catheter

31 Move Catheter

32 Imaging

33 Direction Indicated

34 Calculation

35 Energise Coils + Move

36 Imaging

37 Steer Again — Y

N

38 Move

39 Imaging

40 Correct Position — N

Y

41 Further Procedure

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9911189 A
- WO 9923946 A